(19) Europäisches Patentamt / European Patent Office / Office européen des brevets

(11) **EP 3 139 163 A1**

(12) **EUROPEAN PATENT APPLICATION**
published in accordance with Art. 153(4) EPC

(43) Date of publication:
08.03.2017 Bulletin 2017/10

(21) Application number: 15785971.1

(22) Date of filing: 28.04.2015

(51) Int Cl.:
*G01N 33/543* (2006.01)    *G01N 33/48* (2006.01)
*G01N 33/53* (2006.01)    *G01N 33/72* (2006.01)

(86) International application number:
PCT/JP2015/062906

(87) International publication number:
WO 2015/166969 (05.11.2015 Gazette 2015/44)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA

(30) Priority: 30.04.2014 JP 2014093844

(71) Applicant: Tanaka Kikinzoku Kogyo K.K.
Chiyoda-ku
Tokyo 100-6422 (JP)

(72) Inventors:
• OKAMOTO Koji
Hiratsuka-shi
Kanagawa 254-0076 (JP)

• KATO Yuya
Hiratsuka-shi
Kanagawa 254-0076 (JP)
• ITO Daisuke
Hiratsuka-shi
Kanagawa 254-0076 (JP)
• MIYATA Aki
Hiratsuka-shi
Kanagawa 254-0076 (JP)
• NAKAJIMA Satoru
Hiratsuka-shi
Kanagawa 254-0076 (JP)

(74) Representative: Peguet, Wilfried
Ipsilon
Le Centralis
63, avenue du Général Leclerc
92340 Bourg-la-Reine (FR)

(54) **IMMUNOCHROMOTOGRAPHIC ANALYSIS KIT, IMMUNOCHROMOTOGRAPHIC ANALYSIS DEVICE, AND IMMUNOCHROMOTOGRAPHIC ANALYSIS METHOD**

(57) An object is to provide an immunochromatographic analysis kit, with which detection with high sensitivity is realized even in the case of a blood analyte containing saliva collected from the inside of an oral cavity, and an immunochromatographic analysis can be performed without pain or stress. The above object was achieved by an immunochromatographic analysis kit wherein an alkyl glucoside and an anionic surfactant are included in at least one of an analyte dilution solution and a part upstream in the development direction of a detection part.

[FIG.1]

**Description**

TECHNICAL FIELD

The present invention relates to an immunochromatographic analysis kit, an immunochromatographic analysis device, and an immunochromatographic analysis method.

BACKGROUND ART

Measurement of various components contained in an analyte such as blood or urine is clinically extremely important for ascertaining the physical conditions of a patient, and conventionally, various measurement methods are adopted according to the components. As one of the methods, there has been known an immunochromatographic analysis device and analysis method, in which a detection target contained in an analyte is caused to develop color by an immune reaction, and the developed color signal is confirmed.

The simplest structure of the immunochromatographic analysis device is a structure in which a sample addition part, a labeling substance retaining part, a chromatography medium having a detection part supported thereon, and an absorption part are mutually connected to each other.

On the other hand, there were 366 million diabetic patients in the world in 2011, and the number of diabetic patients in the world is expected to reach 552 million (about 10% of the adult population) in 2030. Further, the number of so-called prediabetic individuals is considered to be equal to or more than the number of diabetic patients.

Conventionally, the diagnosis of diabetes has been made by measuring the blood glucose level. However, recently, the concentration of glycated hemoglobin (glycohemoglobin) in which a sugar is bound to hemoglobin in the blood, particularly hemoglobin A1c (hereinafter referred to as "HbA1c") in which the N-terminal valine residue of the hemoglobin β chain has been glycated with respect to the amount of total hemoglobin reflects the average blood glucose level in the past 1 to 2 months, and therefore has begun to be used as an index suitable for diagnosis of diabetes or follow-up observation of diabetes.

The measurement of HbA1c is conventionally performed by an HPLC method, a capillary electrophoresis method, an enzymatic method, an immunological measurement method, or the like, however, these methods require expert knowledge of a specific device or analysis, and therefore have a problem that the value of HbA1c cannot be easily known in small-sized hospitals, at home, etc.

Further, with respect to the blood level of HbA1c, there are individual differences in blood components, and therefore, a positive or negative determination of diabetes is made by simultaneously measuring hemoglobin other than HbA1c, and ascertaining the ratio of the amount of HbA1c to the amount of total hemoglobin including HbA1c and hemoglobin other than HbA1c. However, in this case, it is necessary to separately measure HbA1c and hemoglobin other than HbA1c by the above-mentioned method, and therefore, there is also a problem that the complexity of the measurement is further increased.

In view of this, for example, Patent Document 1 has proposed a method for simply measuring HbA1c. In the method, first, collected blood and a component for exposing the N-terminal of the hemoglobin β chain on the surface of a protein (N-terminal exposure agent) are mixed, whereby an epitope of HbA1c is exposed on the surface of a hemoglobin protein and a sample solution is prepared, and thereafter, the sample solution is dropped onto a sample addition part. The sample solution is developed on an antibody-immobilized membrane by a capillary phenomenon, and the sample solution developed on the antibody-immobilized membrane reaches an anti-HbA1c antibody-coated part, and only HbA1c in the sample solution reacts in the part and is detected.

Subsequently, the sample solution further developed on the antibody-immobilized membrane reaches an anti-HbA0 antibody-coated part, and only HbA0 in the sample solution reacts and is detected, and the other forms of hemoglobin do not react and migrate to a water absorption pad.

In this manner, HbA0 and HbA1c in the sample solution are detected respectively. This method does not require expert knowledge of a specific device or analysis unlike an HPLC method, a capillary electrophoresis method, an enzymatic method, an immunological measurement method, or the like, and therefore, the value of HbA1c can be easily known.

CITED REFERENCES

PATENT DOCUMENT

Patent Document 1: JP-A-2012-251789

DISCLOSURE OF THE INVENTION

PROBLEMS TO BE SOLVED BY THE INVENTION

However, the method described in Patent Document 1 is a method for measuring HbA1c in a blood analyte, and therefore, the method has a problem that the analyte is inevitably derived from finger blood or the like, and a needle is used every time when blood is collected, and pain or mental stress is caused each time.

A saliva analyte which can be collected without using a needle enables measurement of a substance for which sensitivity as required for hemoglobin is not relatively required, however, it is difficult to measure a very small amount of a substance for which high sensitivity is required such as HbA1c. Further, there is also a problem that in an immunochromatographic analysis, the sensitivity is further decreased due to the presence of a saliva component.

In view of this, an object of the present invention is to solve the above-mentioned problems and provide an immunochromatographic analysis kit, an immunochromatographic analysis device, and an immunochromatographic analysis method capable of performing an immunochromatographic analysis without pain or stress by achieving detection with high sensitivity even in the case of a blood analyte including impurities such as saliva obtained by collecting a small amount of a blood component from the inside of an oral cavity without using a needle.

MEANS FOR SOLVING THE PROBLEMS

As a result of intensive studies, the present inventors found that an immunochromatographic analysis can be performed without pain or stress by including an alkyl glucoside and an anionic surfactant in a part upstream in the development direction of a detection part in the immunochromatographic analysis so as to achieve detection with high sensitivity even in the case of a blood analyte containing impurities such as saliva collected from the inside of an oral cavity, and thus, the present invention could be completed.

That is, the present invention is as follows.

1. An immunochromatographic analysis kit, comprising an analyte dilution solution for diluting and developing a blood analyte collected from the inside of an oral cavity, and an immunochromatographic analysis device which includes a sample addition part, a labeling substance retaining part, a chromatography medium part having a detection part supported thereon, and an absorption part.

2. The immunochromatographic analysis kit as described in 1 above, wherein an alkyl glucoside and an anionic surfactant are included in at least one of the analyte dilution solution and a part upstream in the development direction of the detection part.

3. The immunochromatographic analysis kit as described in 1 or 2 above, wherein a detection target in the blood analyte is a glycated protein in blood.

4. The immunochromatographic analysis kit as described in 3 above, wherein the glycated protein is HbA1c.

5. The immunochromatographic analysis kit as described in any one of 2 to 4 above, wherein the alkyl glucoside is an alkyl glucoside or an alkyl maltoside having an alkyl group with 5 to 15 carbon atoms.

6. The immunochromatographic analysis kit as described in any one of 2 to 5 above, wherein the anionic surfactant is at least one member selected from the group consisting of sodium dodecyl sulfonate, sodium dodecylbenzene sulfonate, and sodium polyoxyethylene lauryl ether sulfate.

7. The immunochromatographic analysis kit as described in any one of 2 to 6 above, wherein in the part upstream in the development direction of the detection part, the alkyl glucoside in an amount of 1 to 1700 $\mu$g and the anionic surfactant in an amount of 8 to 800 $\mu$g are included.

8. The immunochromatographic analysis kit as described in any one of 1 to 7 above, wherein the blood analyte is collected from the inside of the oral cavity using an interdental brush.

9. An immunochromatographic analysis device, which is an immunochromatographic analysis device for detecting a detection target in a blood analyte collected from the inside of an oral cavity, comprising a sample addition part, a labeling substance retaining part, a chromatography medium part having a detection part supported thereon, and an absorption part, wherein an alkyl glucoside and an anionic surfactant are included in a part upstream in the development direction of the detection part.

10. An immunochromatographic analysis method, which is a method for detecting a detection target contained in a blood analyte collected from the inside of an oral cavity using an immunochromatographic analysis device which includes a sample addition part, a labeling substance retaining part, a chromatography medium part having a detection part supported thereon, and an absorption part, comprising the following steps (1) to (4):

(1) a step of adding an analyte-treated solution obtained by diluting the blood analyte with an analyte dilution solution to the sample addition part;

(2) a step of recognizing the detection target by a labeling substance retained in the labeling substance retaining part;

(3) a step of developing the blood analyte and the labeling substance in the chromatography medium part as a mobile phase in the presence of an alkyl glucoside and an anionic surfactant; and

(4) a step of detecting the detection target in the developed mobile phase in the detection part.

EFFECT OF THE INVENTION

The immunochromatographic analysis kit, the immunochromatographic analysis device, and the immunochromatographic analysis method of the present invention are configured such that an alkyl glucoside is included in at least one of an analyte dilution solution and a part upstream in the development direction of a detection part, and therefore, before a detection target reaches the detection part, the detection target comes in contact with the alkyl glucoside to stabilize the detection target in a blood analyte containing saliva collected from the inside of an oral cavity, and thus, the detection target can be detected with high sensitivity.

Further, inclusion of an anionic surfactant in at least one of the analyte dilution solution and the part upstream in the development direction of the detection part along with the alkyl glucoside can contribute to suppression of the decrease in the detection reaction reactivity due to a saliva component.

BRIEF DESCRIPTION OF DRAWINGS

[FIG. 1] FIG. 1 (a) and FIG. 1(b) show a structure of an immunochromatographic analysis device.

[FIG. 2] FIG. 2 shows evaluation of the percentage change in the degree of color development for a blood analyte containing saliva (analyte-treated solution 1) and a blood analyte (analyte-treated solution 2).

[FIG. 3] FIG. 3 shows evaluation of the percentage change in the degree of color development for the blood analyte containing saliva (analyte-treated solution 1) and the blood analyte (analyte-treated solution 2).

[FIG. 4] FIG. 4 shows evaluation of the percentage change in the degree of color development for an analyte containing saliva (analyte-treated solution 1') and an analyte free of saliva (analyte-treated solution 2').

EMBODIMENTS FOR CARRYING OUT THE INVENTION

Hereinafter, the present invention will be described in more detail.

The blood analyte to be used in the present invention is, for example, a blood sample such as blood, plasma, or serum collected from the inside of an oral cavity, and is particularly an analyte containing saliva.

Examples of the detection target in the present invention include tumor marker substances such as $\alpha$-fetoprotein (AFP) and carcinoembryonic antigens (CEA), serum proteins such as ferritin, prostate-specific antigens (PSA), and immunoglobulin G (IgG), hormone-related substances such as rheumatoid factors, growth hormone (GH), and adrenocorticotropic hormone (ACTH), influenza viruses, adenoviruses, bacterial antigens such as Chlamydia antigen and Streptococcus pyogenes antigen, and hemoglobin-derived proteins.

Among these, preferred is a detection target in blood, and examples thereof include hemoglobin-derived proteins, plasma proteins, lipoproteins, secretory proteins, hormones, complements, lipids, cholesterol, sugars, other components in the body, drugs administered to the body, and metabolites thereof. Above all, from the viewpoint of the effect of the present invention, the detection target is preferably glycated proteins such as glycated albumin and glycated hemoglobin in blood, and is more preferably HbA1c.

Hereinafter, a description will be made by showing an example in which HbA1c is used as a detection target, and hemoglobin other than HbA1c is used as a substance to serve as a standard for making a positive or negative determination, however, the present invention is not limited to the following example.

The immunochromatographic analysis kit of the present invention includes an analyte dilution solution for diluting and developing a blood analyte containing saliva, and an immunochromatographic analysis device which includes a sample addition part, a labeling substance retaining part, a chromatography medium part having a detection part supported thereon, and an absorption part.

The analyte dilution solution can act as a developing solution for developing a blood analyte and a labeling substance in the chromatography medium part as a mobile phase.

The analyte dilution solution preferably includes a nonionic surfactant from the viewpoint that it has favorable developing properties and also does not inhibit the antigen-antibody reaction.

Examples of the nonionic surfactant include polyoxyethylene alkyl ethers, polyoxyethylene/polyoxypropylene alkyl ethers, polyoxyethylene sorbitan fatty acid esters, polyoxyethylene p-t-octylphenyl ethers, polyoxyethylene p-t-nonylphenyl ethers, alkyl polyglucosides, fatty acid diethanolamide, and alkyl monoglyceryl ethers.

In the analyte dilution solution, for example, water is used as a solvent, and the nonionic surfactant can be included in a proportion of, for example, 0.01 to 5 mass%, and is preferably included in a proportion of 0.03 to 3.0 mass%, more preferably included in a proportion of 0.3 to 3.0 mass%. Further, in order to adjust the pH, an additive such as a buffer or an inorganic salt may be added as needed.

It is preferred that the analyte dilution solution is made to include an alkyl glucoside. According to this, a detection target comes in contact with the alkyl glucoside to stabilize the detection target in a blood analyte containing saliva, and thus, the decrease in the sensitivity can be prevented.

Examples of the alkyl glucoside include benzyl-$\alpha$-D-glucoside, benzyl-$\beta$-D-glucoside, octyl-$\alpha$-D-glucoside, octyl-$\beta$-D-glucoside, decyl-$\alpha$-D-glucoside, decyl-$\beta$-D-glucoside, dodecyl-$\alpha$-D-glucoside, dodecyl-$\beta$-D-glucoside, pentadecyl-$\alpha$-D-glucoside, pentadecyl-$\beta$-D-glucoside, benzyl-$\alpha$-D-maltoside, benzyl-$\beta$-D-maltoside, octyl-$\alpha$-D-maltoside, octyl-$\beta$-D-maltoside, decyl-$\alpha$-D-maltoside, decyl-$\beta$-D-maltoside, dodecyl-$\alpha$-D-maltoside, dodecyl-$\beta$-D-maltoside, pentadecyl-$\alpha$-D-maltoside, and pentadecyl-$\beta$-D-maltoside, and the alkyl glucoside is preferably an alkyl glucoside or an alkyl maltoside having an alkyl group with 5 to 15 carbon atoms.

By using such a compound, the solubilization of impurities contained in saliva or the like is promoted, and the decrease in the reactivity between a detection target and a detection substance can be further suppressed, and therefore, the use of such a compound is suitable also from the viewpoint of solubility in a developing component including water. More preferably, an alkyl glucoside having an alkyl group with 7 to 15 carbon atoms is used, and further more preferably, an alkyl glucoside having an alkyl group with 8 to 12 carbon atoms is used.

Above all, octyl-$\beta$-D-glucoside, decyl-$\beta$-D-glucoside, dodecyl-$\beta$-D-glucoside, octyl-$\beta$-D-maltoside, decyl-$\beta$-D-maltoside, or dodecyl-$\beta$-D-maltoside is most preferably used.

The content of the alkyl glucoside in the analyte dilution solution is preferably from 0.025 to 50 mM, more preferably from 0.1 to 20 mM, further more preferably from 5 to 20 mM.

It is because when the content is 0.025 mM or more, the solubilization of a saliva component by the alkyl glucoside is promoted and the decrease in the detection reaction reactivity of a detection target in a blood analyte containing saliva collected from the inside of an oral cavity is suppressed, and when the content is 50 mM or less, detection with high sensitivity can be achieved without causing inhibition of the detection reaction due to an excess concentration of the alkyl glucoside, and further, the concentration is preferred also from the economic viewpoint.

Further, it is preferred that the analyte dilution solution is made to include an anionic surfactant along with the alkyl glucoside. This contributes to suppression of the decrease in the detection reaction reactivity due to a saliva component. In addition, it has a function to expose an epitope of HbA1c in collected blood on the surface of a hemoglobin protein. Specifically, it functions as a component for exposing the N-terminal of the hemoglobin $\beta$ chain on the surface of a protein (N-terminal exposure agent).

Examples of the anionic surfactant include sodium dodecylbenzene sulfonate (SDBS), sodium dodecyl sulfonate (SDS), sodium C12-18 alkyl sulfonate (sodium alkane sulfonate), sodium dialkyl sulfosuccinate, sodium cholate, sodium deoxycholate, and sodium polyoxyethylene lauryl ether sulfate (SLES).

Among these, particularly preferred are sodium dodecyl sulfonate, sodium dodecylbenzene sulfonate, sodium deoxycholate, and sodium polyoxyethylene lauryl ether sulfate. More preferably, sodium dodecyl sulfonate, sodium dodecylbenzene sulfonate, and sodium polyoxyethylene lauryl ether sulfate are used.

The concentration of the anionic surfactant in the analyte dilution solution is preferably from 0.01 to 1.5% (w/v), more preferably from 0.05 to 1.0%, further more preferably from 0.1 to 0.6%.

It is because when the content is 0.01% or more, it can further contribute to suppression of the decrease in the detection reaction reactivity due to a saliva component, and when the content is 1.5% or less, detection with higher sensitivity can be achieved without causing poor development due to aggregation of a labeling reagent or inhibition of the detection reaction due to an excess concentration of the anionic surfactant.

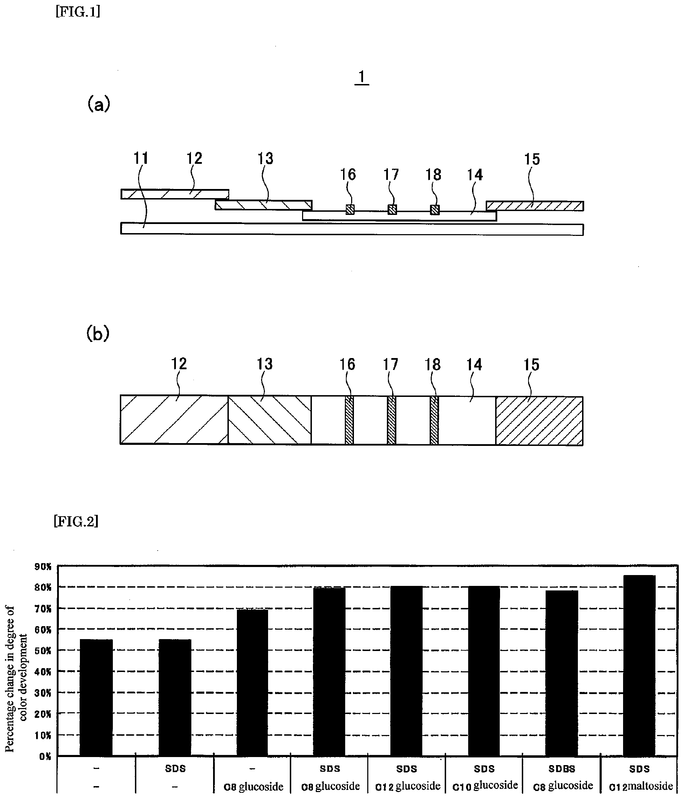

The immunochromatographic analysis device includes a sample addition part, a labeling substance retaining part, a chromatography medium part having a detection part supported thereon, and an absorption part. Hereinafter, the chromatographic analysis device of the present invention will be described with reference to the drawings. FIG. 1(a) and FIG. 1(b) are schematic views for explaining one example of the immunochromatographic analysis device of the present invention, and FIG. 1(a) is a cross-sectional view, and FIG. 1(b) is a plan view.

As shown in FIG. 1(a), an immunochromatographic analysis device 1 includes a sample addition part 12, a labeling substance retaining part 13 including an anti-hemoglobin antibody labeled with a labeling substance, a chromatography medium part 14, and an absorption part 15, which are provided on a plastic adhesive sheet 11 in this order along the longitudinal direction of the kit, respectively.

Further, on the chromatography medium part 14, as detection parts, an anti-HbA1c antibody-coated part 16 coated with an anti-HbA1c antibody, an anti-hemoglobin antibody-coated part 17 coated with an anti-hemoglobin antibody, and an anti-IgG antibody-coated part 18 coated with an anti-IgG antibody as a control are provided, respectively.

The plastic adhesive sheet 11 serves as a base material of the immunochromatographic analysis device 1, and one surface is made to serve as an adhesive surface by coating one surface with an adhesive or by sticking an adhesive tape to one surface, and part or the entire of the respective constituent parts described below are closely adhered onto the adhesive surface. As a material of the plastic adhesive sheet 11, a material which is impermeable to a sample and also is impermeable to moisture may be appropriately selected.

The sample addition part 12 can be composed of a porous sheet having properties such that it quickly absorbs the below-mentioned analyte-treated solution, but has a low ability to retain the analyte-treated solution so that the analyte-treated solution promptly migrates to a region where an antigen-antibody reaction occurs. Examples of the porous sheet include a pad, a fiber, a membrane, etc. composed of glass fiber, cellulose, polyethylene terephthalate, polyurethane, polyacetate, cellulose acetate, nitrocellulose, nylon, a cotton cloth, etc. Above all, the sample addition part 12 is preferably formed using one member selected from the group consisting of glass fiber, cellulose, and polyethylene terephthalate.

At this time, when one member selected from the group consisting of glass fiber, cellulose, and polyethylene terephthalate is used as a material forming the sample addition part 12, the contact efficiency between HbA1c and the anionic surfactant becomes high, and thus, an effect that the anionic surfactant, which will be described in detail later, efficiently acts as the N-terminal exposure agent is exhibited.

It is preferred that the sample addition part 12 is made to include the alkyl glucoside because of the above-mentioned reasons. The content of the alkyl glucoside in the sample addition part 12 is preferably from 1 to 1700 $\mu$g, more preferably from 3.5 to 700 $\mu$g, further more preferably from 175 to 700 $\mu$g.

It is because when the content is 1 $\mu$g or more, the solubilization of a saliva component by the alkyl glucoside is promoted and the decrease in the detection reaction reactivity of a detection target in a blood analyte containing saliva collected from the inside of an oral cavity is suppressed, and when the content is 1700 $\mu$g or less, detection with high sensitivity can be achieved without causing inhibition of the detection reaction due to an excess concentration of the alkyl glucoside, and further, the concentration is preferred also from the economic viewpoint.

Further, in the sample addition part 12, the alkyl glucoside can be included in an amount of 0.5 to 1100 $\mu$g/cm$^2$, and is preferably included in an amount of 2 to 500 $\mu$g/cm$^2$, more preferably included in an amount of 100 to 500 $\mu$g/cm$^2$.

Further, it is preferred that the sample addition part 12 is made to include an anionic surfactant along with the alkyl glucoside because of the above-mentioned reasons. The content of the anionic surfactant in the sample addition part 12 is preferably from 8 to 800 $\mu$g, more preferably from 10 to 500 $\mu$g, further more preferably from 16 to 480 $\mu$g.

It is because when the content is 8 $\mu$g or more, it can further contribute to suppression of the decrease in the detection reaction reactivity due to a saliva component, and when the content is 800 $\mu$g or less, detection with higher sensitivity can be achieved without causing poor development due to aggregation of a labeling reagent or inhibition of the detection reaction due to an excess concentration of the anionic surfactant.

Further, in the sample addition part 12, the anionic surfactant can be included in an amount of 5 to 500 $\mu$g/cm$^2$, and is preferably included in an amount of 6 to 320 $\mu$g/cm$^2$, more preferably included in an amount of 10 to 300 $\mu$g/cm$^2$.

In addition, it is also possible to add any of a variety of additives such as sodium thiocyanate, guanidine hydrochloride, and EDTA to the sample addition part 12 as needed.

The labeling substance retaining part 13 retains, for example, an anti-hemoglobin antibody labeled with a labeling substance. The anti-hemoglobin antibody in the labeling substance retaining part 13 can specifically bind to hemoglobin in an analyte-treated solution. Examples of such an antibody include a polyclonal antibody and a monoclonal antibody. The monoclonal antibody and the polyclonal antibody or fragments thereof are known and available, and can be prepared by a known method.

Examples of an animal species that produces the antibody include a human being, a mouse, a rat, a rabbit, and a goat. The immunoglobulin may be any of IgG, IgM, IgA, IgE, and IgD. The monoclonal antibody is obtained according to a conventional method as follows. The spleen cells and myeloma cells of a mouse immunized with an antigen are fused, and a hybridoma which produces a desired antibody is selected, and a monoclonal antibody produced from this hybridoma is obtained (see, for example, the method of Kohler and Milstein [Nature, 256 (1975), 495-497]). The polyclonal antibody is obtained by separating a desired antibody from an antiserum obtained by immunizing an antibody-producing animal (for example, a human being, a mouse, a rat, a rabbit, a goat, a horse, etc.) with an antigen according to a conventional method.

As the labeling substance, a colored substance capable of visually confirming coloration is preferred, and a substance known in the art can be appropriately adopted. Examples thereof include colloidal metal particles, colloidal non-metal particles, colored latex, and an enzyme label, however, colloidal metal particles whose color hardly fades even if time has passed are particularly preferred from the viewpoint of stability of the label. As the colloidal metal particles, colloidal gold, platinum, copper, silver, and palladium, and other than these, particles obtained by mixing these colloidal metals can be used. In particular, colloidal gold particles are preferred from the viewpoint that the particles having an appropriate particle diameter exhibit red color.

The average particle diameter of the colloidal metal particles is in the range of, for example, 1 to 500 nm, from the viewpoint of obtaining a strong color tone, preferably 10 nm to 150 nm, more preferably 20 to 100 nm. As the colloidal non-metal particles, colloidal selenium and the like can be exemplified. The colloidal metal particles and the colloidal non-metal particles can be prepared according to a conventional method, and at this time, the particle diameter is adjusted so as to exhibit a desired color tone. In addition, it is also possible to use a commercially available product.

Examples of the colored latex include high-molecular weight polymer particles such as polystyrene particles colored with a coloring agent exhibiting red or blue color, and such colored latex can be prepared according to a conventional method. Examples of the enzyme label include peroxidase, alkaline phosphatase, glucose oxidase, and galactosidase. In the case where the enzyme label is used, a substrate for the enzyme and, according to need, a color-developing reagent are allowed to act on the enzyme, and the color developed by the reaction is detected.

Incidentally, the preparation of the antibody labeled with a labeling substance can be performed according to a known method. For example, as a method for supporting colloidal gold particles on the antibody, a known method such as physical adsorption or chemical binding can be employed. Specifically, for example, the antibody is added to a solution in which gold particles are colloidally dispersed to cause a physical adsorption, and thereafter, a blocking protein such as a bovine serum albumin solution is added thereto to block the surface of the particle to which the antibody is not bound, whereby the preparation can be performed. In addition, for the antigen-antibody reaction, a known sandwich method, a competition method, a method combining these methods can be adopted.

Examples of a material of the labeling substance retaining part 13 include glass fiber, cellulose, polyethylene terephthalate, polyurethane, polyacetate, nylon, and a cotton cloth.

It is preferred that the labeling substance retaining part 13 is made to include the alkyl glucoside because of the above-mentioned reasons. The content of the alkyl glucoside in the labeling substance retaining part 13 is preferably from 1 to 1700 $\mu$g, more preferably from 3.5 to 700 $\mu$g, further more preferably from 175 to 700 $\mu$g.

It is because when the content is 1 $\mu$g or more, the solubilization of a saliva component by the alkyl glucoside is promoted and the decrease in the detection reaction reactivity of a detection target in a blood analyte containing saliva collected from the inside of an oral cavity is suppressed, and when the content is 1700 $\mu$g or less, detection with high sensitivity can be achieved without causing inhibition of the detection reaction due to an excess concentration of the alkyl glucoside, and further, the concentration is preferred also from the economic viewpoint.

Further, in the labeling substance retaining part 13, the alkyl glucoside can be included in an amount of 0.5 to 1100 $\mu$g/cm$^2$, and is preferably included in an amount of 2 to 500 $\mu$g/cm$^2$, more preferably included in an amount of 100 to 500 $\mu$g/cm$^2$.

Further, it is preferred that the labeling substance retaining part 13 is made to include an anionic surfactant along with the alkyl glucoside because of the above-mentioned reasons. The content of the anionic surfactant in the labeling substance retaining part 13 is preferably from 8 to 800 $\mu$g, more preferably from 10 to 500 $\mu$g, further more preferably from 16 to 480 $\mu$g.

It is because when the content is 8 $\mu$g or more, it can further contribute to suppression of the decrease in the detection reaction reactivity due to a saliva component, and when the content is 800 $\mu$g or less, detection with higher sensitivity can be achieved without causing poor development due to aggregation of a labeling reagent or inhibition of the detection reaction due to an excess concentration of the anionic surfactant.

Further, in the labeling substance retaining part 13, the anionic surfactant can be included in an amount of 5 to 500 $\mu$g/cm$^2$, and is preferably included in an amount of 6 to 320 $\mu$g/cm$^2$, more preferably included in an amount of 10 to 300 $\mu$g/cm$^2$.

The chromatography medium part 14 may be any as long as it can absorb a sample analyte by a capillary phenomenon and allows the sample analyte to migrate therein, and can be composed of, for example, nitrocellulose, cellulose acetate, nylon, polyether sulfone, polyvinyl alcohol, polyester, glass fiber, polyolefin, cellulose, a mixed fiber thereof, or the like.

The anti-HbA1c antibody-coated part 16, the anti-hemoglobin antibody-coated part 17, and the anti-IgG antibody-coated part 18 in the detection part provided on the chromatography medium part 14 are composed of, for example, a material capable of supporting and fixing each antibody, and examples of the material include nitrocellulose.

It is preferred that the chromatography medium part 14 is made to include the alkyl glucoside because of the above-mentioned reasons. The content of the alkyl glucoside in the chromatography medium part 14 is preferably from 1 to 1700 $\mu$g, more preferably from 3.5 to 700 $\mu$g, further more preferably from 175 to 700 $\mu$g.

It is because when the content is 1 $\mu$g or more, the solubilization of a saliva component by the alkyl glucoside is promoted and the decrease in the detection reaction reactivity of a detection target in a blood analyte containing saliva collected from the inside of an oral cavity is suppressed, and when the content is 1700 $\mu$g or less, detection with high sensitivity can be achieved without causing inhibition of the detection reaction due to an excess concentration of the alkyl glucoside, and further, the concentration is preferred also from the economic viewpoint.

Further, in the chromatography medium part 14, the alkyl glucoside can be included in an amount of 0.5 to 1100 $\mu$g/cm$^2$, and is preferably included in an amount of 2 to 500 $\mu$g/cm$^2$, more preferably included in an amount of 100 to 500 $\mu$g/cm$^2$.

Further, it is preferred that the chromatography medium part 14 is made to include an anionic surfactant along with the alkyl glucoside because of the above-mentioned reasons. The content of the anionic surfactant in the chromatography medium part 14 is preferably from 8 to 800 $\mu$g, more preferably from 10 to 500 $\mu$g, further more preferably from 16 to 480 $\mu$g.

It is because when the content is 8 $\mu$g or more, it can further contribute to suppression of the decrease in the detection reaction reactivity due to a saliva component, and when the content is 800 $\mu$g or less, detection with higher sensitivity can be achieved without causing poor development due to aggregation of a labeling reagent or inhibition of the detection reaction due to an excess concentration of the anionic surfactant.

Further, in the chromatography medium part 14, the anionic surfactant can be included in an amount of 5 to 500 $\mu$g/cm$^2$, and is preferably included in an amount of 6 to 320 $\mu$g/cm$^2$, more preferably included in an amount of 10 to 300 $\mu$g/cm$^2$.

In the immunochromatographic analysis device of the present invention, in a part upstream in the development direction of the detection part described above, the above-mentioned alkyl glucoside and anionic surfactant are included. Here, the "upstream in the development direction of the detection part" as used herein refers to a relative direction when a sample addition part side is defined as an upstream side when the detection part is used as a reference in the longitudinal direction of the immunochromatographic analysis device. Specifically, for example, the sample addition part 12, the labeling substance retaining part 13, chromatography medium part 14, and the like can be exemplified.

It is preferred that the alkyl glucoside and the anionic surfactant are included in at least one of the analyte dilution solution, and the sample addition part 12, the labeling substance retaining part 13, and the chromatography medium part 14 located upstream in the development direction of the detection part as described above. From the viewpoint of effective suppression of the decrease in the detection reaction reactivity due to a saliva component or suppression of poor development due to aggregation of a labeling reagent, it is more preferred that the alkyl glucoside and the anionic surfactant are included in at least one of the analyte dilution solution and the sample addition part. It is particularly preferred that the alkyl glucoside is included in the analyte dilution solution, and the anionic surfactant is included in the sample addition part.

Examples of a material constituting the absorption part 15 include a material having an ability to quickly absorb an excess analyte-treated solution, and cellulose fiber, a glass filter paper, or the like is used.

Next, the immunochromatographic analysis method of the present invention will be described. The immunochromatographic analysis method of the present invention is a method for detecting a detection target contained in a blood analyte collected from the inside of an oral cavity using the immunochromatographic analysis device described above, and includes the following steps (1) to (4):

(1) a step of adding an analyte-treated solution obtained by diluting the blood analyte with an analyte dilution solution to the sample addition part;
(2) a step of recognizing the detection target by a labeling substance retained in the labeling substance retaining part;
(3) a step of developing the blood analyte and the labeling substance in the chromatography medium part as a mobile phase in the presence of an alkyl glucoside and an anionic surfactant; and
(4) a step of detecting the detection target in the developed mobile phase in the detection part.

Hereinafter, the respective steps will be described showing a case where the detection target is HbA1c as an example. In the step (1), first, an analyte-treated solution is prepared by diluting a blood analyte collected from the inside of an oral cavity with an analyte dilution solution. The analyte dilution solution can act as a developing solution for developing a blood analyte and a labeling substance in the chromatography medium part as a mobile phase in the step (3).

The analyte dilution solution is mixed with a blood analyte collected from the inside of an oral cavity of a patient, whereby an analyte-treated solution is prepared and dropped onto the sample addition part 12. Examples of a site where the blood analyte is collected from a patient include a gingival sulcus. Further, examples of the collection method include a method using an interdental brush.

In the step (2), hemoglobin in the analyte-treated solution having reached the labeling substance retaining part 13 is subjected to an antigen-antibody reaction with the anti-hemoglobin antibody labeled with a labeling substance, whereby a complex is formed. That is, HbA1c, which is the detection target, is also recognized by the labeling substance retained in the labeling substance retaining part 13.

In the step (3), the analyte and the labeling substance, that is, the analyte-treated solution and the complex of the anti-hemoglobin antibody and hemoglobin are developed in the chromatography medium part 14 as a mobile phase. At this time, the nonionic surfactant included in the analyte-treated solution exhibits an effect that the developing properties in the development are improved and also the subsequent antigen-antibody reaction is not inhibited as described above.

Further, in order to develop the analyte and the labeling substance in the chromatograph medium part as the mobile phase in the presence of an alkyl glucoside and an anionic surfactant, the alkyl glucoside and the anionic surfactant are included in at least one of the analyte dilution solution and a part upstream in the development direction of the detection part.

Subsequently, in the step (4), HbA1c, which is the detection target in the mobile phase developed in the chromatography medium part 14, reaches the anti-HbA1c antibody-coated part 16, and HbA1c reacts with the anti-HbA1c antibody while it is passing through the part and is immobilized thereon. Hemoglobin other than HbA1c and the analyte-treated solution do not react and pass through the anti-HbA1c antibody-coated part 16, however, when reaching the anti-hemoglobin antibody-coated part 17, hemoglobin other than HbA1c reacts with the anti-hemoglobin antibody and is immobilized thereon.

Incidentally, the labeling substance which does not react with the antigen or the labeling substance which does not react in the antibody-coated parts 16 and 17 reacts with the anti-IgG antibody in the anti-IgG antibody-coated part 18 and is immobilized thereon, and develops color as a control showing that the development is performed normally. The other components of the sample solution do not react and migrate to the absorption part 15. In this manner, the developed color signals due to the presence of HbA1c and hemoglobin other than HbA1c can be confirmed in the respective coated parts. Incidentally, the anti-HbA1c antibody and the anti-hemoglobin antibody may be either a monoclonal antibody or a polyclonal antibody as described above.

There are individual differences in blood components, and it is difficult to make the determination of diabetes only by causing HbA1c to develop color and confirming the strength of the developed color. Therefore, it is preferred that HbA1c and hemoglobin other than HbA1c are caused to simultaneously develop color and the determination is made by comparison between the degrees of color development of HbA1c and hemoglobin other than HbA1c.

Specifically, for example, the developed color signals of HbA1c and hemoglobin other than HbA1c are compared, and in the case where the developed color signal of HbA1c is stronger than that of hemoglobin other than HbA1c, a positive determination can be made. On the other hand, in the case where the developed color signal of HbA1c is equal to or weaker than that of hemoglobin other than HbA1c, a negative determination can be made.

EXAMPLES

Hereinafter, the present invention will be further described by way of Examples and Comparative Examples, however, the present invention is not limited to the following examples.

<Test Example 1>

In Test Example 1, an effect that an alkyl glucoside and an anionic surfactant contribute to suppression of the decrease in the detection reaction reactivity due to a saliva component was confirmed by using "a glycated protein (HbA1c) in a blood analyte containing saliva" as a detection target and including the alkyl glucoside in an analyte dilution solution and including and the anionic surfactant in a sample addition part.

Example 1

An immunochromatographic analysis device 1 as shown in FIGS. 1(a) and 1(b) was prepared according to the following procedure.

(1) Sample addition part 12

A sample addition part 12 was prepared by uniformly adding sodium dodecyl sulfate (SDS) in an amount of 60 $\mu$g/cm$^2$ to a glass fiber pad (manufactured by Millipore, Inc., trade name: Glass Fiber Conjugate Pad, size: length 32 mm (in the development direction of the analyte-treated solution), width 150 mm, thickness 0.43 mm), followed by drying at 50°C for 4 hours.

(2) Preparation of anti-HbA1c antibody-coated part 16, anti-hemoglobin antibody-coated part 17, and anti-IgG antibody-coated part 18

As a membrane, a sheet composed of nitrocellulose (manufactured by Millipore, Inc., trade name: HF 120, 250 mm $\times$ 25 mm) was used. An anti-HbA1c monoclonal antibody, an anti-hemoglobin monoclonal antibody, or an anti-IgG monoclonal antibody was diluted to a concentration of 1.0 mg/ml with a 10 mM phosphate buffer solution (pH 7.4) including 5 mass% sucrose and 5 mass% isopropanol. The diluted solutions (150 $\mu$L) were applied to different places with a width of 1 mm on the membrane by an antibody applicator (manufactured by BioDot, Inc.), followed by drying at 50°C for 30 minutes and then drying at room temperature overnight, whereby an anti-HbA1c antibody-coated part 16, an anti-hemoglobin antibody-coated part 17, and an anti-IgG antibody-coated part 18 were provided, respectively, on a chromatography medium part 14.

(3) Preparation of labeling substance solution

To 0.5 mL of a colloidal gold suspension (manufactured by Tanaka Kikinzoku Kogyo K.K., average particle diameter: 40 nm), 0.1 mL of a solution in which an anti-hemoglobin monoclonal antibody was diluted to a concentration of 0.1 mg/mL with Tris buffer (pH 8.5) was added, and the resulting mixture was left to stand at room temperature for 10 minutes. Then, 0.1 mL of a Tris buffer solution (pH 8.5) including 0.01 mass% PEG-SH (manufactured by NOF Corporation, trade name: SUNBRIGHT ME-200SH, molecular weight: 20,000) was added thereto (PEG-SH concentration after addition: 0.001 mass%), and the resulting mixture was left to stand at room temperature for 10 minutes. Thereafter, the mixture was thoroughly stirred, and then centrifuged at 8000 $\times$ g for 15 minutes. After removing the supernatant, 0.1 mL of a phosphate buffer solution (pH 7.4) including 1 mass% bovine serum albumin was added thereto, whereby a labeling substance solution was prepared.

(4) Preparation of Immunochromatographic analysis device 1

A solution obtained by adding 100 $\mu$L of a phosphate buffer solution (pH 9.0) including a 25 mass% aqueous solution of trehalose to 220 $\mu$L of the labeling substance solution prepared above was added uniformly to a 8 mm $\times$ 100 mm glass fiber pad (manufactured by Millipore, Inc.), followed by drying in a vacuum dryer, whereby a labeling substance retaining part 13 was prepared. Subsequently, on a plastic adhesive sheet 11, the sample addition part 12, the labeling substance retaining part 13 labeled with a labeling substance, and the chromatography medium part 14 prepared above were stuck, and a general-purpose absorption part 15 was further stuck. Then, the resulting material was cut to a width of 5 mm by a cutter, whereby an immunochromatographic analysis device 1 was prepared. At this time, the amount of SDS included per device was 96 $\mu$g.
An analyte dilution solution was prepared by stirring the respective components according to the following formulation.

> As a glucoside, C8 glucoside (manufactured by DOJINDO Laboratories, product name: n-octyl-β-D-glucoside): 10 mM
> As a nonionic surfactant, a mixture of Triton X-100 (trade name, manufactured by SIGMA, Inc.) and Tween 20 (trade name, manufactured by Wako Pure Chemical Industries, Ltd.) at a mass ratio of 1:5: 1.2 mass%
> As a buffer, a Bicine buffer solution: 50 mM
> As an inorganic salt, potassium chloride: 0.6 mass%
> As an additive, casein sodium: 2.0 mass%
> Balance: water

Collection of analyte

Blood having an HbA1c concentration of 5.5% was prepared by mixing blood having a known HbA1c concentration collected by pricking the finger of each of healthy adult males and diabetic male patients. Further, saliva was collected from healthy adult males.

Preparation of analyte-treated solution 1

Blood and saliva were mixed at 1:49 (volume ratio), and the resulting mixture and the analyte dilution solution were mixed at 1:19 (former : latter (volume ratio)), whereby an analyte-treated solution 1 was prepared.

Preparation of analyte-treated solution 2

Blood which did not contain saliva and the analyte dilution solution were mixed at 1:1000 (former : latter (volume ratio)), whereby an analyte-treated solution 2 was prepared.

Preparation of analyte-treated solution 3

Blood (1 $\mu$L) having an HbA1c concentration of 6.5% prepared by mixing blood having a known HbA1c concentration collected by pricking the finger of each of healthy adult males and diabetic male patients and a blood analyte containing saliva collected from the inside of the oral cavity of healthy adult males by pricking a gingival sulcus with an interdental brush were immersed in 1.0 mL of the analyte dilution solution, followed by stirring, whereby an analyte-treated solution 3 was prepared.

Implementation of immunochromatographic analysis

To the sample addition part 12 of the immunochromatographic analysis device 1 prepared as described above, 110 μL of the analyte-treated solution 1 was supplied, and 10 minutes after development, the degree of color development in the detection part was measured using an immunochromato-reader (product name, manufactured by Hamamatsu Photonics K.K.). As for the measurement value using the immunochromato-reader, the test was performed repeatedly 3 times for the respective Examples and Comparative Examples.

An immunochromatographic analysis was performed also for the analyte-treated solution 2 in the same manner.

Evaluation of percentage change in degree of color development for blood analyte containing saliva (analyte-treated solution 1) and blood analyte (analyte-treated solution 2)

The percentage change in the degree of color development obtained according to the following formula is shown in Table 1 and FIG. 2.

$$\text{Percentage change in degree of color development (\%)} = 100 \times \frac{\text{(average measurement value of analyte-treated solution 1)}}{\text{(average measurement value of analyte-treated solution 2)}}$$

"100%" indicates "no change", and a smaller numerical value indicates a larger decrease in the sensitivity to the analyte containing saliva.

Example 2

The procedure of Example 1 was repeated except that the alkyl glucoside in the analyte dilution solution in Example 1 was changed from C8 glucoside to C12 glucoside, n-dodecyl-β-D-glucoside (manufactured by Kao Corporation, product name: MYDOL 12). The result is shown in Table 1 and FIG. 2.

Example 3

The procedure of Example 1 was repeated except that the alkyl glucoside in the analyte dilution solution in Example 1 was changed from C8 glucoside to C10 glucoside, n-decyl-β-D-glucoside (manufactured by Kao Corporation, product name: MYDOL 10). The result is shown in Table 1 and FIG. 2.

Example 4

The procedure of Example 1 was repeated except that the anionic surfactant included in the sample addition part in Example 1 was changed from SDS to SDBS (manufactured by Kanto Chemical Co., Inc., product name: sodium dodecylbenzene sulfonate). The result is shown in Table 1 and FIG. 2.

Example 5

The procedure of Example 1 was repeated except that the alkyl glucoside in the analyte dilution solution in Example 1 was changed from C8 glucoside to C12 maltoside (manufactured by DOJINDO Laboratories, product name: n-Dodecyl-β-D-maltoside). The result is shown in Table 1 and FIG. 2.

Comparative Example 1

The procedure of Example 1 was repeated except that the alkyl glucoside in the analyte dilution solution and SDS in the sample addition part in Example 1 were not included. The result is shown in Table 1 and FIG. 2.

Comparative Example 2

The procedure of Example 1 was repeated except that C8 glucoside in the analyte dilution solution in Example 1 was not included. The result is shown in Table 1 and FIG. 2.

Comparative Example 3

The procedure of Example 1 was repeated except that SDS in the sample addition part in Example 1 was not included. The result is shown in Table 1 and FIG. 2.

Comparative Example 4

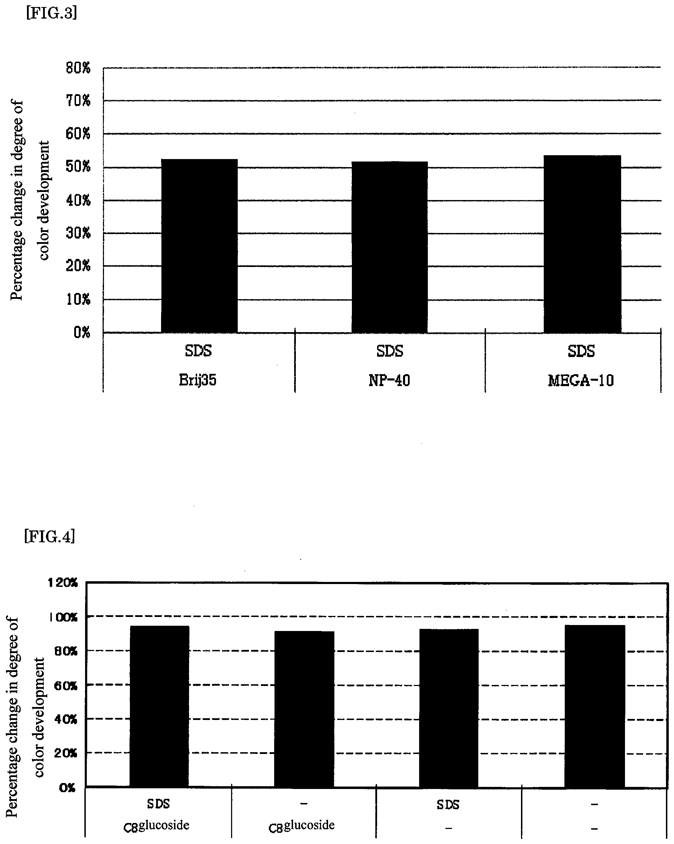

The procedure of Example 1 was repeated except that in place of C8 glucoside in the analyte dilution solution in Example 1, Brij 35 (manufactured by ICI Americas, Inc.), which is a nonionic surfactant similarly to C8 glucoside, was included at 10 mM. The result is shown in Table 2 and FIG. 3.

Comparative Example 5

The procedure of Example 1 was repeated except that in place of C8 glucoside in the analyte dilution solution in Example 1, NP-40 (manufactured by Nacalai Tesque, Inc., trade name: Nonidet (registered trademark) P-40), which is a nonionic surfactant similarly to C8 glucoside, was included at 10 mM. The result is shown in Table 2 and FIG. 3.

Comparative Example 6

The procedure of Example 1 was repeated except that in place of C8 glucoside in the analyte dilution solution in Example 1, MEGA-10 (manufactured by DOJINDO Laboratories, N-decanoyl-N-methyl-D-glucamine), which is a nonionic surfactant similarly to C8 glucoside, was included at 10 mM. The result is shown in Table 2 and FIG. 3.

[Table 1]

Table 1

|  |  | Comparative Example 1 | Comparative Example 2 | Comparative Example 3 | Example 1 | Example 2 | Example 3 | Example 4 | Example 5 |
|---|---|---|---|---|---|---|---|---|---|
| Analyte dilution solution | Glucoside | - | - | C8 glucoside | C8 glucoside | C12 glucoside | C10 glucoside | C8 glucoside | C12 maltoside |
| Sample addition part | Anionic surfactant | - | SDS | - | SDS | SDS | SDS | SDBS | SDS |
| Percentage change in degree of color development | | 55% | 55% | 69% | 79% | 80% | 80% | 78% | 85% |

[Table 2]

Table 2

| | | Comparative Example 4 | Comparative Example 5 | Comparative Example 6 |
|---|---|---|---|---|
| Analyte dilution solution | Nonionic surfactant | Brij35 | NP-40 | MEGA-10 |
| Sample addition part | Anionic surfactant | SDS | SDS | SDS |
| Percentage change in degree of color development | | 53% | 52% | 53% |

From the results of Examples shown in Table 1 and FIG. 2, it is found that by including an alkyl glucoside and an anionic surfactant in a part upstream in the development direction of the detection part, the decrease in the detection sensitivity due to the inclusion of saliva in a blood analyte is suppressed.

Further, in the case where an alkyl glucoside is not included in Comparative Examples 1 and 2, there is no difference in the percentage change in the degree of color development whether or not an anionic surfactant is included.

On the other hand, it is found that in the case where an alkyl glucoside is included in Comparative Example 3 and Example 1, the decrease in the detection sensitivity is suppressed more in the case where an anionic surfactant is included.

Therefore, it was found that by the presence of both of an alkyl glucoside and an anionic surfactant, a synergistic effect is exhibited, and the decrease in the detection sensitivity to a blood analyte due to the inclusion of saliva is suppressed.

Further, the results of Comparative Examples 4 to 6 shown in Table 2 and FIG. 3 show that in the case where Brij 35, NP-40, or MEGA-10, which is a nonionic surfactant similarly to an alkyl glucoside is included in place of the alkyl glucoside in Example 1, the percentage change in the degree of color development significantly decreased as compared with Example 1. From these results, it was revealed that in the present invention, by using particularly an alkyl glucoside among the nonionic surfactants, even a detection target in a blood analyte containing saliva can be detected with remarkably high sensitivity.

<Test Example 2>

In Test Example 2, a test was performed by changing the detection target in Test Example 1 to a substance other than "a glycated protein in a blood analyte containing saliva", and it was confirmed that a marked effect characteristic of the present invention is obtained by using "a glycated protein in a blood analyte containing saliva" as a detection target. In this test, the test was performed by using an adenovirus antigen as a detection target.

Comparative Example 7

In Comparative Example 7, an immunochromatographic analysis device was prepared according to the following procedure.

(1) A sample addition part was prepared in the same manner as in Example 1.

(2) Preparation of detection part

As a membrane, a sheet composed of nitrocellulose (manufactured by Millipore, Inc., trade name: HF 120, 250 mm $\times$ 25 mm) was used. An anti-adenovirus monoclonal antibody or an anti-IgG monoclonal antibody was diluted to a concentration of 1.0 mg/ml with a 10 mM phosphate buffer solution (pH 7.4) including 5 mass% sucrose and 5 mass% isopropanol. The diluted solutions (150 $\mu$L) were applied to different places with a width of 1 mm on the membrane by an antibody applicator (manufactured by BioDot, Inc.), followed by drying at 50°C for 30 minutes and then drying at room temperature overnight, whereby an anti-adenovirus monoclonal antibody-coated part and an anti-IgG antibody-coated part were provided, respectively, on a chromatography medium part.

(3) Preparation of labeling substance solution

To 0.5 mL of a colloidal gold suspension (manufactured by Tanaka Kikinzoku Kogyo K.K., average particle diameter: 40 nm), 0.1 mL of a solution in which the anti-adenovirus monoclonal antibody was diluted to a concentration of 0.1 mg/mL with Tris buffer (pH 8.5) was added, and the resulting mixture was left to stand at room temperature for 10 minutes. Then, 0.1 mL of a Tris buffer solution (pH 8.5) including 0.01 mass% PEG-SH (manufactured by NOF Corporation, trade name: SUNBRIGHT ME-200SH, molecular weight: 20,000) was added thereto (PEG-SH concentration after addition: 0.001 mass%), and the resulting mixture was left to stand at room temperature for 10 minutes. Thereafter, the mixture was thoroughly stirred, and then centrifuged at 8000 $\times$ g for 15 minutes. After removing the supernatant, 0.1 mL of a phosphate buffer solution (pH 7.4) including 1 mass% bovine serum albumin was added thereto, whereby a labeling substance solution was prepared.

(4) Preparation of Immunochromatographic analysis device

A solution obtained by adding 100 $\mu$L of a phosphate buffer solution (pH 9.0) including a 25 mass% aqueous solution of trehalose to 220 $\mu$L of the labeling substance solution prepared above was added uniformly to a 8 mm $\times$ 100 mm glass fiber pad (manufactured by Millipore, Inc.), followed by drying in a vacuum dryer, whereby a labeling substance retaining part was prepared. Subsequently, on a plastic adhesive sheet, the sample addition part, the labeling substance retaining part labeled with a labeling substance, and the chromatography medium part prepared above were stuck, and a general-purpose absorption part was further stuck. Then, the resulting material was cut to a width of 5 mm by a cutter, whereby an immunochromatographic analysis device was prepared. At this time, the amount of SDS included per device was 96 $\mu$g.

An analyte dilution solution was prepared by stirring the respective components according to the following formulation.

As a glucoside, C8 glucoside (manufactured by DOJINDO Laboratories, product name: n-octyl-$\beta$-D-glucoside): 10 mM
As a nonionic surfactant, a mixture of Triton X-100 (trade name, manufactured by SIGMA, Inc.) and Tween 20 (trade name, manufactured by Wako Pure Chemical Industries, Ltd.) at a mass ratio of 1:5: 1.2 mass%
As a buffer, a Bicine buffer solution: 50 mM
As an inorganic salt, potassium chloride: 0.6 mass%
As an additive, casein sodium: 2.0 mass%
Balance: water

Collection of analyte

As a detection substance, 1 $\mu$g/mL of an inactivated adenovirus antigen was used, and as for saliva, saliva was collected from healthy adult males.

Preparation of analyte-treated solution 1'

The inactivated adenovirus antigen and saliva were mixed at 1:49 (volume ratio), and the resulting mixture and the analyte dilution solution were mixed at 1:19 (former : latter (volume ratio)), whereby an analyte-treated solution 1' was prepared.

Preparation of analyte-treated solution 2'

The inactivated adenovirus antigen and the analyte dilution solution were mixed at 1:1000 (former : latter (volume ratio)), whereby an analyte-treated solution 2' was prepared.

Implementation of immunochromatographic analysis

To the sample addition part of the immunochromatographic analysis device prepared as described above, 110 $\mu$L of the analyte-treated solution 1' was supplied, and 10 minutes after development, the degree of color development was measured using an immunochromato-reader (product name, manufactured by Hamamatsu Photonics K.K.). As for the measurement value using the immunochromato-reader, the test was performed repeatedly 3 times for the respective Comparative Examples.

An immunochromatographic analysis was performed also for the analyte-treated solution 2' in the same manner as described above.

Evaluation of percentage change in degree of color development for adenovirus analyte containing saliva (analyte-treated solution 1') and adenovirus analyte free of saliva (analyte-treated solution 2')

The percentage change in the degree of color development obtained according to the following formula is shown in Table 3 and FIG. 4.

$$\text{Percentage change in degree of color development (\%)} = 100 \times \text{(average measurement value of analyte-treated solution 1') / (average measurement value of analyte-treated solution 2')}$$

"100%" indicates "no change", and a smaller numerical value indicates a larger decrease in the sensitivity to the analyte containing saliva.

Comparative Example 8

The procedure of Comparative Example 7 was repeated except that SDS in the sample addition part in Comparative Example 7 was not included. The result is shown in Table 3 and FIG. 4.

Comparative Example 9

The procedure of Comparative Example 7 was repeated except that the alkyl glucoside in the analyte dilution solution in Comparative Example 7 was not included. The result is shown in Table 3 and FIG. 4.

Comparative Example 10

The procedure of Comparative Example 7 was repeated except that the alkyl glucoside in the analyte dilution solution and SDS in the sample addition part in Comparative Example 7 were not included. The result is shown in Table 3 and FIG. 4.

[Table 3]

Table 3

|  |  | Comparative Example 7 | Comparative Example 8 | Comparative Example 9 | Comparative Example 10 |
|---|---|---|---|---|---|
| Analyte dilution solution | Glucoside | C8 glucoside | C8 glucoside | - | - |
| Sample addition part | Anionic surfactant | SDS | - | SDS | - |
| Percentage change in degree of color development | | 95% | 91% | 93% | 96% |

The results of Comparative Examples 7 to 10 shown in Table 3 and FIG. 4 show that in the case where an adenovirus was used as a detection target, even if an alkyl glucoside or an anionic surfactant was included in the analyte-treated solution, a difference in the percentage change in the degree of color development was almost not observed. Therefore, it was found that in the present invention, an effect of improvement of the sensitivity to a detection target is obtained by using "a glycated protein in a blood analyte containing saliva" as the detection target.

Example 6

The procedure of Example 1 was repeated except that in the preparation of the analyte-treated solution 1 in Example 1, blood having various HbA1c concentrations of 5.5%, 6.0%, and 6.5% was used, and only an analysis of blood analytes containing saliva was performed. Further, in an immunochromatographic analysis, the degree of color development in the detection part was determined by visual observation without using an immunochromato-reader. The results are shown in Table 4. Incidentally, the evaluation criteria in the table are as follows.

+: The development of red color can be confirmed.
++: The development of strong red color can be confirmed.
+++: The development of very strong red color can be confirmed.

[Table 4]

Table 4

| Concentration of HbA1c | 5.5% | 6.0% | 6.5% |
|---|---|---|---|
| Visual determination | + | ++ | +++ |

Example 7

The procedure of Example 6 was repeated except that a test was performed for an actual analyte of blood containing saliva by using an analyte-treated solution 3 in place of the analyte-treated solution 1 in Example 6. As a result of visual determination, the development of very strong red color (+++) was confirmed. Further, the same result is obtained also in the case where a test is performed by collecting a blood analyte of a diabetic patient having an HbA1c concentration of 6.5% or more using an interdental brush, and treating the analyte by 1000-fold dilution with the analyte dilution solution.

While the present invention has been described in detail with reference to specific embodiments, it is apparent to those skilled in the art that various changes and modifications can be made without departing from the spirit and scope of the present invention. The present application is based on Japanese Patent Application (Japanese Patent Application No. 2014-093844) filed on April 30, 2014 and the entire contents of which are incorporated herein by reference.

REFERENCE SIGNS LIST

1    immunochromatographic analysis device

11    plastic adhesive sheet

12    sample addition part

13    labeling substance retaining part

14    chromatography medium part

15    absorption part

16    anti-HbA1c antibody-coated part coated with anti-HbA1c antibody

17    anti-hemoglobin antibody-coated part coated with anti-hemoglobin antibody

18    anti-IgG antibody-coated part coated with anti-IgG antibody as control

**Claims**

1.  An immunochromatographic analysis kit, which is an immunochromatographic analysis kit comprising an analyte dilution solution for diluting and developing a blood analyte containing saliva collected from the inside of an oral cavity, and an immunochromatographic analysis device which includes a sample addition part, a labeling substance retaining part, a chromatography medium part having a detection part supported thereon, and an absorption part, wherein

a detection target in the blood analyte is a glycated protein in blood,

the analyte dilution solution includes an alkyl glucoside, and

an anionic surfactant is included in at least one of the analyte dilution solution and a part upstream in the development direction of the detection part.

2. The immunochromatographic analysis kit according to claim 1, wherein the sample addition part includes an anionic surfactant.

3. The immunochromatographic analysis kit according to claim 1 or 2, wherein the glycated protein is HbA1c.

4. The immunochromatographic analysis kit according to any one of claims 1 to 3, wherein the alkyl glucoside is an alkyl glucoside or an alkyl maltoside having an alkyl group with 5 to 15 carbon atoms.

5. The immunochromatographic analysis kit according to any one of claims 1 to 4, wherein the anionic surfactant is at least one member selected from the group consisting of sodium dodecyl sulfonate, sodium dodecylbenzene sulfonate, and sodium polyoxyethylene lauryl ether sulfate.

6. The immunochromatographic analysis kit according to any one of claims 1 to 5, wherein the analyte dilution solution includes the alkyl glucoside in an amount of 0.025 to 50 mM, and the part upstream in the development direction of the detection part includes the anionic surfactant in an amount of 8 to 800 $\mu$g.

7. The immunochromatographic analysis kit according to any one of claims 1 to 6, wherein the blood analyte is collected from the inside of the oral cavity using an interdental brush.

8. An immunochromatographic analysis method, which is a method for detecting a detection target contained in a blood analyte containing saliva collected from the inside of an oral cavity using an immunochromatographic analysis device which includes a sample addition part, a labeling substance retaining part, a chromatography medium part having a detection part supported thereon, and an absorption part, wherein a detection target in the blood analyte is a glycated protein in blood, and the method comprises the following steps (1) to (4):

(1) a step of adding an analyte-treated solution obtained by diluting the blood analyte with an analyte dilution solution including an alkyl glucoside to the sample addition part;
(2) a step of recognizing the detection target by a labeling substance retained in the labeling substance retaining part;
(3) a step of developing the blood analyte and the labeling substance in the chromatography medium part as a mobile phase in the presence of an alkyl glucoside and an anionic surfactant; and
(4) a step of detecting the detection target in the developed mobile phase in the detection part.

9. The immunochromatographic analysis method according to claim 8, wherein the sample addition part includes an anionic surfactant.

[FIG.1]

(a)

<u>1</u>

(b)

[FIG.2]

[FIG.3]

[FIG.4]

## INTERNATIONAL SEARCH REPORT

| International application No. |
| --- |
| PCT/JP2015/062906 |

| A. CLASSIFICATION OF SUBJECT MATTER |
| --- |
| *G01N33/543*(2006.01)i, *G01N33/48*(2006.01)i, *G01N33/53*(2006.01)i, *G01N33/72* (2006.01)i |

According to International Patent Classification (IPC) or to both national classification and IPC

| B. FIELDS SEARCHED |
| --- |
| Minimum documentation searched (classification system followed by classification symbols)<br>G01N33/543, G01N33/48, G01N33/53, G01N33/72 |

| Documentation searched other than minimum documentation to the extent that such documents are included in the fields searched |
| --- |
| Jitsuyo Shinan Koho          1922–1996   Jitsuyo Shinan Toroku Koho   1996–2015<br>Kokai Jitsuyo Shinan Koho    1971–2015   Toroku Jitsuyo Shinan Koho   1994–2015 |

| Electronic data base consulted during the international search (name of data base and, where practicable, search terms used) |
| --- |

### C. DOCUMENTS CONSIDERED TO BE RELEVANT

| Category* | Citation of document, with indication, where appropriate, of the relevant passages | Relevant to claim No. |
| --- | --- | --- |
| X/Y | JP 2013-195403 A  (Tanaka Kikinzoku Kogyo Kabushiki Kaisha), 30 September 2013 (30.09.2013), entire text; all drawings; particularly, paragraphs [0029], [0030], [0036], [0038]; fig. 1 <br> & JP 2013-195403 A       & US 2015/0118675 A1 <br> & WO 2013/141122 A1      & EP 2829879 A1 <br> & TW 201400815 A         & CN 104204802 A <br> & KR 10-2014-0137367 A | 1,2,5,6,9,10 /3,4,7,8 |
| X/Y | WO 2013/073558 A1  (Rohto Pharmaceutical Co., Ltd.), 23 May 2013 (23.05.2013), entire text; all drawings; particularly, paragraphs [0003], [0014], [0015]; fig. 1 <br> & JP 2013-873558 A | 1,2,5,6,9,10 /3,4,7,8 |

☒  Further documents are listed in the continuation of Box C.        ☐  See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered   to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 03 July 2015 (03.07.15) | 14 July 2015 (14.07.15) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
| --- | --- |

Form PCT/ISA/210 (second sheet) (July 2009)

**INTERNATIONAL SEARCH REPORT**

| International application No. |
|---|
| PCT/JP2015/062906 |

C (Continuation).   DOCUMENTS CONSIDERED TO BE RELEVANT

| Category* | Citation of document, with indication, where appropriate, of the relevant passages | Relevant to claim No. |
|---|---|---|
| X/Y | JP 2009-052945 A  (Sunstar Inc.) 12 March 2009 (12.03.2009), entire text; all drawings; particularly, paragraphs [0011], [0020]; fig. 1 (Family: none) | 1,2,5,6,9,10 /3,4,7,8 |
| Y | JP 2012-251789 A  (Sekisui Medical Co., Ltd.), 20 December 2012 (20.12.2012), entire text; all drawings (Family: none) | 3,4,7,8 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012251789 A

- JP 2014093844 A

**Non-patent literature cited in the description**

- **KOHLER ; MILSTEIN.** *Nature,* 1975, vol. 256, 495-497